(19) Europäisches Patentamt
European Patent Office
Office européen des brevets

(11) EP 1 774 971 A1

(12) EUROPEAN PATENT APPLICATION

(43) Date of publication:
**18.04.2007 Bulletin 2007/16**

(21) Application number: **05380228.6**

(22) Date of filing: **14.10.2005**

(51) Int Cl.:
*A61K 31/722* (2006.01) *A61K 31/727* (2006.01)
*A61K 33/42* (2006.01) *A61K 9/51* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Advanced in Vitro Cell Technologies, S.L.**
**08028 Barcelona (ES)**

(72) Inventors:
- **Vila Pena, Ana**
  **15782 Santiago de Compostela (ES)**
- **Suárez Luque, Silvia**
  **15782 Santiago de Compostela (ES)**
- **Alonso Fernández, María José**
  **15782 Santiago de Compostela (ES)**

(74) Representative: **Bernardo Noriega, Francisco**
**Orense, 68, 7th floor**
**28020 Madrid (ES)**

# (54) Chitosan and heparin nanoparticles

(57) The invention is aimed at nanoparticulate systems for the controlled release of heparin through mucosa. It is specifically aimed at nanoparticulate systems comprising chitosan, heparin and optionally a polyoxyethylenated derivative, and which are ionically cross-linked, as well as being aimed at processes for obtaining them.

EP 1 774 971 A1

## Description

### FIELD OF THE INVENTION

The invention is aimed at nanoparticulate systems for controlled release of heparin through mucosa. It is particularly aimed at nanoparticulate systems comprising chitosan, heparin and optionally a polyoxyethylenated derivative, and which are ionically crosslinked, as well as processes for obtaining them.

### STATE OF THE ART

The systems for releasing biologically active agents form a constantly developing field of research. It is known that there are drawbacks to the administration of active ingredients to the human and animal body through different administration routes. Some drugs, amongst which are peptides, proteins and polysaccharides, are not effectively absorbed through mucosal surfaces due to the limited permeability of the epithelial barriers in the human and animal body. One example of these drugs having little capacity to cross mucosal barriers is heparin, the administration of which is currently by parenteral or subcutaneous route, making it therefore necessary to develop administration systems allowing a better absorption of this active molecule through mucosae if alternative routes to those currently existing want to be found. It is also especially desirable that they may be administered orally.

There are documents which describe biocompatible and biodegradable polymers, such as chitosan, combined with heparin, and their pharmaceutical use.

Thus, document EP0771206 describes the use of a matrix of chitosan and heparin immobilised thereto by precipitation or by covalent bonds, for manufacturing a drug capable of regenerating hard tissue, such as bone tissue. The combination may be in the form of powder, solution, film or gel.

Patent EP0930885 also refers to the use of heparin in combination with chitosan for obtaining a drug which prevents infections caused by the herpes virus. It may be in any physical form, such as a suspension, solution or gel.

Patents EP0772446 and EP0759760 describe the use of chitosan and a polysaccharide, such as heparin immobilised thereto by means of ionic or covalent bonds or by mechanical inclusion, in order to regenerate tissues in the case of wounds. The composition may be in the form of a film, membrane, tube, solution, powder or gel.

None of these documents mentions a system in the form of nanoparticles or its use for controlled administration through mucosa.

On the other hand, patent application WO03/090763 is aimed at the use of an aqueous composition comprising chitosan complexes in combination with heparin for rectal treatment of inflammatory diseases by topical administration of the solution.

Patent application WO96/20730 refers to a pharmaceutical formulation comprising a chitosan with a specific degree of acetylation and molecular weight as a polymer allowing increasing the epithelial permeability of hydrophilic drugs. Low molecular weight heparin is mentioned amongst other active agents as a possible therapeutic agent.

The incorporation of active ingredients in small particles is to be emphasised amongst the recently proposed possibilities for overcoming the biological barriers confronted by drugs.

In this sense, patent applications WO98/04244, WO2004/009060 and WO2004/11275 describe nanoparticulate systems containing chitosan for the administration of active ingredients.

Patent application WO96/05810 describes chitosan particles for administration on mucosa, specifying the addition of a low molecular weight heparin solution to already formed chitosan microspheres of sizes between 10-50 microns, forming a suspension which is frozen and lyophilised for its administration.

Andersson, M. and Lofroth, JE describe in Int J Pharm. 2003, 257(1-2) 305-309 nanometric-size particles of a heparin/chitosan complex which are formed with microemulsions.

Despite the numerous publications aimed at developing heparin release systems, there is still a great need for providing a type of small size particulate system of easy production and with high yields having a great capacity for association with heparin and which allows release thereof through mucosa at a controlled rate.

### BRIEF DESCRIPTION OF THE INVENTION

With the present invention the inventors have found that a system made up of nanoparticles comprising chitosan and heparin, in the presence or absence of a polyoxyethylenated derivative, and obtained by means of an ionic gelling process in the presence of an agent causing chitosan crosslinking, allows an effective heparin molecule association as well as the subsequent release thereof in a suitable biological environment. Surprisingly, these nanoparticles are stable in gastrointestinal fluids and present an excellent effectiveness and bioavailability, such as demonstrated by the data obtained *in vivo.* The release occurs in a controlled and slow manner. These systems are therefore very adequate for oral administration. In fact, heparin plasma levels have been obtained by means of the oral administration of these

nanoparticles up to 10 times greater than when heparin is administered in a solution.

Furthermore, the nanoparticulate systems proposed in the invention for association and controlled release of heparin have numerous advantages such as (1) the heparin incorporation process is simple and does not require the use of toxic ingredients for the organism; (2) their physicochemical properties, specifically their size and surface charge, can be modulated according to the ratio of formulation components and their molecular weight; (3) they have an extraordinary heparin association capacity; and (4) they release said active molecule at a controlled rate.

Therefore, an object of the present invention consists in a pharmaceutical composition comprising nanoparticles with a size of less than 1 micron for the controlled release of heparin through mucosa, where the nanoparticles comprise at least chitosan or a derivative thereof, and at least one heparin or derivative thereof, and where the nanoparticles are crosslinked by means of a crosslinking agent.

In a preferred variant of the invention the crosslinking agent is a polyphosphate salt, preferably sodium tripolyphosphate.

In another variant of the invention the nanoparticles comprise:

a) between 50% and 90% by weight of chitosan or a derivative thereof, and

b) between 10% and 50% by weight of heparin or a derivative thereof.

In another variant of the system, the chitosan-heparin nanoparticles further comprise a polyoxyethylenated compound, preferably a polyoxyethylene or an ethylene oxide-propylene oxide copolymer.

In a preferred variant of the invention the nanoparticles present a positive charge, preferably a Z potential between +1 and +40 mV.

In a preferred aspect, the composition is for oral administration.

Another aspect of the invention consists in a process for preparing a pharmaceutical composition for controlled release of heparin through mucosa such as that defined above and comprising:

a) preparing an aqueous solution comprising chitosan or a derivative thereof;

b) preparing an aqueous solution comprising heparin or a derivative thereof and the crosslinking agent; and

c) mixing, with stirring, the solutions of steps a) and b), such that the chitosan-heparin nanoparticles are spontaneously obtained by means of ionic gelling.

## DETAILED DESCRIPTION OF THE DRAWINGS

Figure 1 shows low molecular weight heparin (LMWH) plasma levels expressed as anti-Xa activity (IU/ml) after oral administration of LMWH in solution and associated to high molecular weight chitosan (HMWCS) nanoparticles in rats (n=5).

Figure 2 shows low molecular weight heparin (LMWH) plasma levels expressed as anti-Xa activity (IU/ml) after oral administration of LMWH in solution and associated to low molecular weight chitosan (LMWCS) nanoparticles in rats (n=5).

## DETAILED DESCRIPTION OF THE INVENTION

Chitosan is a natural polymer which has an aminopolysaccharide structure and a cationic character. It comprises the repetition of monomer units of formula (1):

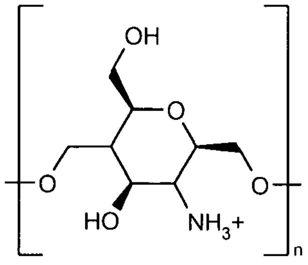

(I)

where n is an integer and represents the degree of polymerisation, i.e. the number of monomer units in the chitosan chain.

In addition to these monomer units, chitosan generally contains a proportion of monomer units in which the amino group is acetylated. In fact, chitosan is obtained by deacetylation of chitin (100% acetylated). Said degree of deacetylation is generally within a range comprised between 30 and 95, preferably between 55 and 90, which indicates that between 10 and 45% of the amino groups are acetylated.

The chitosan used to obtain the nanoparticles of the present invention has a molecular weight comprised between 2 and 2000 kDa, preferably between 2 and 500 kDa, more preferably between 5 and 150 kDa.

In a variant of the invention the so-called low molecular weight chitosan (LMWCS) is used, in which said molecular weight is less than 10 kDa. In another variant the so-called high molecular weight chitosan (HMWCS) is used, in which its molecular weight ranges between 100 and 150 kDa.

As an alternative to chitosan a derivative thereof may be used in the nanoparticles of the present invention, understanding as such a chitosan in which one or more hydroxyl or amino groups have been modified. These derivatives include, amongst others, O-acetylated, O-alkylated or O-sulfonated chitosans, such as are described in Chitin Chemistry, Macmillan, 1992, 166.

Several covalent derivatives of chitosan have been synthesised to date, such as trimethylchitosan, the object of which has been to increase chitosan solubility in mediums with a pH above 6.5. There are likewise thiolated derivatives of chitosan, produced with the object of increasing the mucoadhesive character of chitosan. In both cases, funcionalisation of chitosan has been performed through the amino group.

For its part, heparin is a natural substance in the blood, a polysaccharide involved in the blood clotting process. Its chemical structure comprises the repetition of monomer units of formula (II):

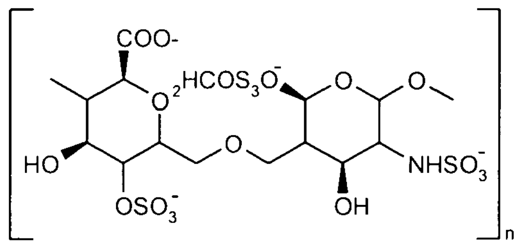

(II)

where n is an integer and represents the degree of polymerisation, i.e. the number of monomer units in the heparin chain.

Traditional or unfractionated heparin (UFH) is distinguished from fractionated or low molecular weight heparin (LMWH). The first one is a natural substance, present in all vertebrates. It is formed by multiple chains of variable molecular weights, which gives it great heterogeneity, however, all the chains are integrated by the combination of two sugars: uronic acid and glucosaline. Chain length varies, although it may be established that it has an average of 50 sugars per chain, with a mean molecular weight of 15 kDa. It is used as is or preferably in the form of a salt, such as for example its sodium or calcium salt.

Fractionated or low molecular weight heparin is produced by chemical or enzymatic depolymerisation of conventional heparins. Examples of this type of heparins are enoxaparin, parnaparin, dalteparin and nadroparin, and their

salts such as their sodium and calcium salts. In general, chains having 18 sugars, or 5.4 kDa, are obtained, in a large proportion. Under this length the effects of heparin change from the enzymatic point of view, as do their pharmacokinetics.

Heparin derivatives may also be used instead of unfractionated or low molecular weight heparin in the composition of the present invention. These derivatives are known and originate from the reactivity of the different functional groups present in the molecules, as can be seen in formula II. Thus N-desulfated, N-acetylated, O-decarboxylated heparins, oxidised or reduced heparins, etc., are known.

Amongst the known applications of heparins or their derivatives are the prevention and treatment of deep venous thrombosis, pulmonary, arterial or cerebral thromboembolism, the prevention of clots in patients subjected to surgery, dialysis, or a blood transfusion, due to their anticoagulant activity.

The composition of the invention, comprising nanoparticles for the administration of heparin or derivatives thereof, preferably has a chitosan or a chitosan derivative content comprised between 50 and 99% by weight, preferably between 50% and 90% by weight. On the other hand, the heparin content in the system is preferably comprised between 10 and 50% by weight, preferably between 25% and 40% by weight.

The chitosan-heparin nanoparticulate system of the invention is characterised in that it has been formed by means of a joint precipitation process of chitosan and heparin in the form of polymeric nanoclusters caused by the addition of a crosslinking agent. The use of organic solvents or extreme pH conditions or toxic auxiliary substances is not required. The association of heparin to the nanoparticles occurs according to an ionic interaction mechanism. Heparin has numerous negative groups in its structure, sulphate and carboxylate groups, which justifies a high ionic affinity for the positive amino groups of chitosan, favouring nanoparticle appearance. The presence of the crosslinking agent allows crosslinking of the chitosan-heparin system so that a lattice is formed between which the heparin is inserted which may subsequently be released. Furthermore, the crosslinking agent provides the nanoparticles with their size, potential and structural characteristics which make them suitable as an administration system for said active molecule.

The crosslinking agent is preferably an anionic salt allowing reticulation of the chitosan-heparin system by means of ionic gelling, causing the spontaneous formation of nanoparticles. Preferably a polyphosphate salt is used, sodium tripolyphosphate (TPP) being especially preferred.

In a variant of the system of the invention, the nanoparticles may include a polyoxyethylenated compound. A polyoxyethylenated compound is understood as a synthetic hydrophilic polymer of non-ionic character having units of ethylene oxide in its structure, the use of a polyoxyethylene or an ethylene oxide-propylene oxide (PEO-PPO) copolymer, commonly called poloxamers, being preferred. These polymers are marketed with different molecular weights, however, in the present invention the use of polyoxyethylenated derivatives having molecular weights comprised between 2000 and 10000 is preferred. In a preferred embodiment, the polyoxyethylenated derivative is a triblock copolymer (PEO-PPO-PEO), such as for example that commercially called Poloxamer 188.

The proportion of chitosan with respect to the polyoxyethylenated compound which is incorporated to the particle formation medium may be very variable, ranging between 50:0 and 1:100, preferably between 50:0 and 1:20. The presence of the polyoxyethylenated compound may have as an effect slightly increasing the size of the nanoparticles and it aids in stabilising the colloidal system. If it is added subsequently to nanoparticle formation it forms a coating which significantly increases the size thereof. Although the presence of the polyoxyethylenated derivative is not necessary for obtaining the nanoparticles, it allows modifying the physicochemical characteristics of said nanoparticles (size and zeta potential), and above all, stabilising the system in gastrointestinal fluids.

The preparation and formation of chitosan nanoparticles and an active ingredient, by means of a crosslinking agent in the presence or absence of a polyoxyethylenated compound, is described in WO98/04244, which is herein considered incorporated in its entirety by reference.

The nanoparticles described in the present invention are characterised by having a mean particle size of less than 1 μm, preferably having a mean size comprised between 1 and 999 nm, preferably between 50 and 800 nm, and even more preferably between 50 nm and 500 nm. The mean particle size is mainly influenced by the molecular weight of chitosan, by the degree of deacetylation of chitosan, by the proportion of chitosan with respect to the polyoxyethylenated derivative if present, and also by the particle formation conditions (chitosan concentration, crosslinking agent concentration and ratio between them). In general, the presence of the polyoxyethylenated derivative causes an increase in the mean particle size with respect to systems formed by chitosan without said derivative.

On the other hand, the nanoparticles may present an electric charge (measured by means of the Z potential) attributed to the amino groups of the chitosan, the magnitude of which may range from 0 mV up to +50 mV. It is preferably positive, between 1 and +40 mV, depending on the mentioned variables and particularly on the degree of deacetylation and on the presence or absence of inorganic salts. The positive charge of the nanoparticles may be of interest in order to favour the interaction thereof with mucosal surfaces. However, the neutral charge may be interesting in the case of parenteral administration thereof.

The pharmaceutical composition of the invention may be presented in liquid (nanoparticle suspension) or solid form. In this case the nanoparticles may be found in lyophilised or spray forms, forming a powder which may be used to make granulates, tablets, capsules or preparations for inhalation.

Although the composition is mainly for administration through mucosae, the pharmaceutical composition of the invention may be administered by oral, buccal or sublingual, transdermal, ocular, nasal, vaginal or parenteral route. In the case of the non-parenteral routes the contact of the nanoparticles with the skin or mucosae can be improved by providing the particles with an important positive charge, which will favour their interaction with said negatively charged surfaces.

In a preferred aspect, the formulation is administered by mucosal route. The positive charge of chitosan provides a better absorption of heparin on the mucosal surface through its interaction with the mucosa and the surfaces of the epithelial cells which are negatively charged.

In the case of oral administration they have the additional advantage that they are stable in gastrointestinal fluids, so they may reach and remain without degradation on the intestinal epithelial tissue so as to release the heparin.

The pharmaceutical compositions of the invention are especially suitable, among others, for the prevention of venous thrombosis in surgical patients subjected to orthopaedic surgery or general surgery and in immobilised non-surgical patients, whose situation may be defined as a moderate or high risk situation. They are also indicated in the prevention of clotting in the extracorporeal circulation circuit in haemodialysis, in treating established deep venous thrombosis (with or without pulmonary embolism) and in treating unstable angina and myocardial infarction, jointly administered with other anticoagulants.

Another aspect of the present invention refers to a process for preparing chitosan-heparin nanoparticles such as those previously defined, comprising:

a) preparing an aqueous solution of chitosan or a derivative thereof;

b) preparing an aqueous solution of heparin and of the crosslinking agent; and

c) mixing, with stirring, the solutions of steps a) and b), such that the chitosan-heparin nanoparticles are spontaneously obtained by means of ionic gelling and subsequent precipitation.

In a particular embodiment of the aforementioned process, the resulting crosslinking agent/chitosan ratio is comprised between 0.01/1 and 0.50/1, the 0.05/1 and 0.40/1 ratio being preferred, which provides formulations with a relatively low polydispersity. However, the use of larger or smaller crosslinking agent/chitosan ratios is also possible.

In a variant of the process, it further comprises incorporating the polyoxyethylenated compound to the aforementioned aqueous solution of chitosan.

In another variant of the process for obtaining the nanoparticles of the invention inorganic salts may be used, such as for example sodium chloride, which allow increasing the nanoparticle production yield. They may be added to the chitosan or chitosan derivative solution. However, it has been observed that the addition of said salts modifies the interaction between chitosan and heparin, causing a slight decrease in the amount of heparin associated to the nanoparticulate system, as well as neutralising the nanoparticle charge because the negative ions of the medium are absorbed into the positive surface of the chitosan. Taking this into account, a person skilled in the art may use said salts, according to the desired final characteristics, in the case of low yields, and in amounts leading to a suitable yield but without negatively affecting the degree of association or the charge if this is desired to be sufficiently high as to improve interactions with the mucosa.

In yet another variant of the process, if it is desired that the chitosan-heparin nanoparticles are to be coated by the polyoxyethylenated compound, the latter is incorporated after nanoparticle formation.

These processes for preparing the nanoparticles of the invention allow an association of heparin to the nanoparticles exceeding 90%. Slight variations in said association can be observed depending on the molecular weight of the chitosan, its degree of deacetylation and the presence of the polyoxyethylenated derivative or inorganic salts.

Once the nanoparticles are formed, they may be isolated by means of centrifugation in a glycerol or glucose bed, or in a trehalose solution, discarding the supernatant, with the object of separating the heparin molecules which are not associated to the nanoparticles. The nanoparticles can subsequently be resuspended in water or buffer for their use in suspension.

The process for preparing the chitosan-heparin nanoparticles can further comprise an additional step in which said nanoparticles are lyophilised or atomised. From a pharmaceutical point of view it is important to be able to have the nanoparticles available in lyophilised form since this improves their stability during storage. The chitosan-heparin nanoparticles (in which chitosan may be physically mixed with a polyoxyethylenated derivative) may be lyophilised in the presence of a cryoprotectant, such as glucose, sucrose or trehalose, at a 5% concentration. In fact, the nanoparticles of the invention have the additional advantage that the particle size before and after lyophilisation is not significantly modified. That is, the nanoparticles can be lyophilised and resuspended without an alteration in the characteristics thereof. Likewise, said nanoparticles may also be atomised by the use of mannitol or lactose as adjuvants.

Some illustrative examples are described below which show the features and advantages of the invention; they

should not be interpreted as limiting on the object of the invention.

## EXAMPLES

As a common process to all the examples detailed below, the nanoparticles are characterised from the point of view of size, zeta potential (or surface charge), association effectiveness (percent of heparin that has been associated to the nanoparticles) and production yield (percent of materials forming the nanoparticles).

The *Size Distribution* has been performed by means of photon correlation spectroscopy (PCS; Zeta Sizer, Nano series, Nano-ZS, Malvern Instruments, UK), giving mean size and nanoparticle population dispersion (polydispersity index) values.

The *Zeta Potential* has been measured by Laser Doppler Anemometry (LDA; Zeta Sizer, Nano series, Nano-ZS, Malvern Instruments, UK). The samples were diluted in Milli-Q water so as to determine electrophoretic mobility.

*Quantification of the amount of associated heparin* is indirectly determined by quantifying the heparin that has not been associated, and the latter is in turn determined by quantifying the anti-Xa activity, using a Stachrom® Heparin Kit (Diagnostica Stago, Roche). In order to perform this quantification, the nanoparticles are isolated by centrifugation and the supernatant obtained is filtered through PVDF 0.22 $\mu$m.

The *Production yield* was quantified by means of nanoparticle centrifugation at 16000 xg for 40 minutes, subsequent removal of the supernatant (where the components remaining in solution will be) and finally, weighing the dry residue.

The chitosan (Protasan UP Cl 113) used in the examples comes from NovaMatrix-FMC Biopolymer, the low molecular weight heparin from Aventis (Enoxaparin), Poloxamer 188 from BASF Corporation, and the remaining products used are from Sigma Aldrich, such as unfractionated heparin, sodium tripolyphosphate, sodium chloride, sucrose, glucose, mannitol and salts for preparing the simulated gastrointestinal fluids without enzymes.

### Example 1

Preparation and characterisation of chitosan nanoparticles with different molecular weight heparins (in the presence or absence of Poloxamer 188 in the chitosan solution)

Aqueous solutions of chitosan (CS) (1 mg/ml) were prepared with and without Poloxamer 188 (4 and 10 mg/ml). These solutions were subjected to magnetic stirring while an aqueous solution of different molecular weight heparin (UFH and LMWH; theoretical load of 25% by weight with respect to chitosan) and sodium tripolyphosphate (TPP, CS/TPP ratio: 1/0.1 - 1/0.4). The volume ratio between both aqueous phases was kept constant, 1 ml of chitosan aqueous solution: 0.2 ml of heparin and crosslinking agent solution.

Incorporation of the poloxamer in the chitosan aqueous phase can cause a slight increase in particle diameter and a slight decrease in the percentage of heparin associated to the nanoparticulate system, independently from the type of heparin (UFH or LMWH), such as can be seen in Table I. In some formulations (especially those which associate with UFH) the presence of the poloxamer may also cause a slight decrease in the surface charge (or zeta potential), although this effect is generally negligible. These modifications (physicochemical characteristics) observed when adding the poloxamer in the chitosan aqueous phase, can be attributed to the fact that during system formation, chains thereof remain trapped in the chitosan and heparin matrix.

The data for the polydispersity index which ranges between 0.20 and 0.30 indicate that, independently from the presence or absence of the poloxamer, this parameter is maintained unaltered, and that therefore the poloxamer does not increase nanoparticle size dispersion.

The weight of the poloxamer was not taken into account in the production yield calculations since the poloxamer is in excess and is eliminated with the supernatant after the centrifugation process. In principle, the presence of the poloxamer in solution can favour system stability (it is a stabilising surfactant commonly used in colloidal systems). Based on the production yields obtained it can be said that poloxamer chains effectively exist in the nanoparticle matrix lattices.

Table I:
Size and potential of the chitosan with heparin nanoparticles (theoretical load of 25% with respect to chitosan) in the presence of different proportions of Poloxamer 188 (n ≥ 3; Mean ± SD).

| Heparin Type | Proportion of poloxamer (with respect to CS) | Size (nm) | Polydispersity Index (P.I.) | Zeta Potential (mV) | Heparin Association (% with respect to initial load) | Production Yield (%) |
|---|---|---|---|---|---|---|
| LMWH | 0 | 225 ± 17 | 0.27 | + 31.0 ± 2.3 | 99 | 45.5 ± 3.3 |
| | 4 | 338 ± 70 | 0.33 | + 32.6 ± 1.4 | n.d. | n.d. |
| | 10 | 311 ± 24 | 0.20 | + 31.5 ± 0.8 | 98 | 54.6 ± 2.8* |
| UFH | 0 | 264 ± 33 | 0.22 | + 41.4 ± 1.4 | 97 | 47.6 ± 5.4 |
| | 4 | 322 ± 21 | 0.25 | + 36.0 ± 0.5 | n.d. | n.d. |
| | 10 | 292 ± 28 | 0.24 | + 34.6 ± 1.4 | 95 | 58.3 ± 4.1 * |

n.d.: not determined; LMWH: Low molecular weight heparin: UFH: Unfractionated heparin
*: Poloxamer weight not taken into account

**Example 2**

Preparation and characterisation of chitosan nanoparticles with different molecular weight heparins and coated with Poloxamer 188

Chitosan (CS) nanoparticles (1 mg/ml) were prepared with both types of heparin (UFH and LMWH) according to the process described in Example 1, and poloxamer 188 (10 mg /1 mg CS) was then added. A theoretical load of 25 and 40% with respect to chitosan was tested with the LMWH and a theoretical load of 25% was tested with the UFH, for the purpose of observing the effect of the resulting poloxamer coating.

The physicochemical characteristics of the nanoparticles are shown in Table II. In comparison with the addition of poloxamer in the chitosan solution (Example 1, Table 1), the size increase is more noticeable when it is added to the nanoparticles after their formation process, especially when the theoretical load of heparin is increased from 25% to 40% (with respect to chitosan). Furthermore, the zeta potential of the nanoparticles experiences a slight decrease when they are coated, especially when the UFH heparin is used. These data show that the poloxamer does coat the nanoparticles.

Table II:
Size and potential of the chitosan with heparin nanoparticles (theoretical load of 25% and/or 40% with respect to chitosan) before and after adding Poloxamer 188 (n ≥ 3; Mean ± SD).

| Heparin Type | Initial amount of heparin (with respect to that of CS, %) | Poloxamer addition (with respect to CS) | Size (nm) | Polydispersity Index (P.I.) | Zeta Potential (mV) |
|---|---|---|---|---|---|
| LMWH | 25 | 0 | 225 ± 17 | 0.27 | + 31.0 ± 2.3 |
| | | 10 | 282 ± 18 0.32 | | + 31.5 ± 0.8 |
| | 40 | 0 | 228 ± 5 | 0.21 | +31.5 ± 0.6 |
| | | 10 | 442 ± 21 | 0.29 | +29.4 ± 0.5 |
| UFH | 25 | 0 | 264 ± 33 | 0.22 | + 41.4 ± 1.4 |
| | | 10 | 311 ± 24 | 0.24 | +37.5 ± 0.6 |

CS; Chitosan; LMWH; Low molecular weight heparin; UFH: Unfractionated heparin

**Example 3**

Preparation and characterisation of chitosan nanoparticles with different molecular weight heparins (with and without sodium chloride in the chitosan solution)

Aqueous solutions of chitosan (CS) (2 mg/ml) were prepared in which volumes ranging between 0 and 1.5 mL of sodium chloride (0.9%) were added. These solutions were subjected to magnetic stirring while an aqueous solution of heparin (UFH or LMWH) (theoretical load of 25% with respect to chitosan) and crosslinking agent, sodium tripolyphosphate (TPP, CS/TPP ratio: 1/0.3-1/0.4), was added to them.

As is shown in Table III, if sodium chloride is added to the aqueous phase of chitosan before preparing the nanoparticles, the physicochemical characteristics of the nanoparticles are not significantly modified. However, a noticeable increase in production yield and a slight decrease in the amount of heparin associated to the nanoparticulate system is observed. This shows that the presence of ions in the initial solutions before preparing the nanoparticles slightly modifies the chitosan-heparin interaction.

The zeta potential of the nanoparticles with sodium chloride gives neutral values (about 0 mV) due to the fact that the negative ions of the medium remain absorbed on the surface of the chitosan nanoparticles, neutralizing the charge thereof.

Table III:

Characteristics of the chitosan-heparin nanoparticles after adding sodium chloride to the chitosan solution (n≥3; Mean ± SD)

| Heparin Type | Additive (in the CS aqueous phase) | Size (nm) | Polydispersity Index (P.I.) | Zeta Potential (mV) | Heparin association effectiveness (%) | Production yield (%) |
|---|---|---|---|---|---|---|
| LMWH | --- | 225 ± 17 | 0.27 | + 31.0 ± 2.3 | 99 | 45.5 ± 3.3 |
| | Sodium chloride | 205 ± 20 | 0.19 | ≈ 0* | 96 | 64.8 ± 1.7 |
| UFH | --- | 264 ± 33 | 0.22 | + 41.4 ± 1.4 | 97 | 47.6 ± 5.4 |
| | Sodium chloride | 263 ± 22 | 0.23 | ≈ 0* | 94 | 66.0 ± 2.1 |

CS: Chitosan; LMWH: Low molecular weight heparin; UFH: Unfractionated heparin;
*: The zeta potential of the nanoparticles is shielded by the ions of the medium

**Example 4**

Preparation and characterisation of chitosan nanoparticles with different molecular weight heparins (with Poloxamer 188 and sodium chloride)

Taking into account the previous examples, in which it was seen how the addition of hydrophilic polymers (such as poloxamer) or of salts (such as sodium chloride) modify the characteristics of the nanoparticles, the objective of this experiment was to study the characteristics of the resulting nanoparticles upon adding both substances in the preparation of the nanoparticles.

As is shown in the following Table, if the poloxamer and sodium chloride are added to the chitosan solution, the resulting nanoparticulate system has a larger particle size (basically due to the presence of the poloxamer) and slightly lower association effectiveness (due to the presence of both components).

Table IV:
Influence of the addition of Poloxamer 188 (10 mg) and/or sodium chloride (0.9%, 1.5 mL) in the preparation of chitosan nanoparticles with unfractionated heparin (UFH) and low molecular weight heparin (LMWH) with a load of 25% (n≥3; Mean ± SD).

| Heparin Type | Additive (in the aqueous phase of CS) | Size (nm) | Polydispersity Index (P.I.) | Association (% with respect to initial load) |
|---|---|---|---|---|
| LMWH | --- | 225 ± 17 | 0.27 | 99 |
| | Poloxamer | 282 ± 18 | 0.32 | 98 |
| | Sodium chloride | 205 ± 20 | 0.19 | 96 |
| | Sodium chloride and Poloxamer | 382 ± 31 | 0.25 | 94 |
| UFH | --- | 264 ± 33 | 0.22 | 97 |
| | Poloxamer | 311 ± 24 | 0.38 | 95 |
| | Sodium chloride | 263 ± 22 | 0.23 | 94 |
| | Sodium chloride and Poloxamer | 323 ± 37 | 0.25 | 93 |

CS: Chitosan; LMWH: Low molecular weight heparin; UFH: Unfractionated heparin

**Example 5**

Preparation and characterisation of chitosan nanoparticles (of different molecular weights) with low molecular weight heparin

The objective of this study was to prepare nanoparticles associated to heparin using chitosans of different molecular weights. Aqueous solutions were prepared of chitosan (CS) (1 mg/ml) with different molecular weights (High molecular weight chitosan: HMWCS: 100-150 kDa and Low molecular weight chitosan: LMWCS: <10kDa). The low molecular weight chitosan was obtained after the fragmentation of the high molecular weight chitosan. In order to carry out the fragmentation process, the CS is dissolved (20 mg/ml) in ultrapure water (Milli-Q) by means of magnetic stirring (2-4 hours) and then 0.1 ml of $NaNO_2$ (0.1 M) is added dropwise to the chitosan solution with stirring. The chitosan solution is left under magnetic stirring overnight.

Nanoparticles were prepared with both the high molecular weight chitosan and with the fractionated (low molecular weight) chitosan following the preparation method described in Example 1 (chitosan nanoparticles and heparin, with and without poloxamer as an additive in the aqueous external phase).

The obtained results are represented in Table V. The low molecular weight chitosan nanoparticles have a particle diameter, polydispersity index, zeta potential and association percentage that are less than the data obtained for the nanoparticles prepared with the high molecular weight chitosan. The presence of poloxamer in the preparation of nanoparticles when the chitosan is low molecular weight does not cause the increase in the mean size of the resulting nanoparticle system (Example 1; Table 1).

Table V:
Influence of the molecular weight of chitosan in the formation of nanoparticles with LMWH (theoretical load of 25% with respect to chitosan) and the presence of Poloxamer 188 (n≥3; Mean ± SD)

| CS Type | Additive (in the aqueous phase of CS) | Size (nm) | Polydispersity index (P.I.) | Zeta Potential (mV) | Association effectiveness (%) | Production yield (%) |
|---|---|---|---|---|---|---|
| HMWCS (100-150 kDa) | --- | 225 ± 17 | 0.27 | + 31.0 ± 2.3 | 99 | 45.5 ± 3.3 |
| | Poloxamer | 282 ± 18 | 0.32 | + 31.5 ± 0.8 | 98 | n.d. |
| LMWCS (<10 kDa) | --- | 163 ± 3 | 0.10 | + 25.9 ± 1.8 | 93 | 48.1 ± 1.0 |
| | Poloxamer | 164 ± 3 | 0.13 3 | + 26.6 ± 0.8 | 92 | n.d. |

CS: Chitosan; LMWCS: Low molecular weight chitosan; HMWCS: High molecular weight chitosan; n.d.: not determined

**Example 6**

Preparation and characterisation of chitosan nanoparticles (with a different degree of acetylation) with low molecular weight heparin

LMWH-chitosan nanoparticles were prepared, the latter with two different degrees of acetylation (10-15% and 35-45%) but with the same molecular weight (PM<10 kDa). The acetylation process of chitosan consisted of adding acetic anhydride (2.5 ml) to a chitosan solution (2 mg/ml; 25 ml) and leaving it under magnetic stirring overnight. Then the chitosan was dialyzed for 24 hours for the purpose of eliminating the acetic anhydride that had not reacted and finally lyophilised. Obtaining the low molecular weight chitosan was carried out as indicated in Example 5. It must be indicated that it was necessary to slightly modify the pH of the acetylated chitosan solution since after its solubilization in water, the resulting pH is 5.8-6.0, and it must be lowered to pH 5.0 in order to replicate the conditions of non-acetylated chitosan.

The nanoparticles were prepared by mixing, under magnetic stirring, the chitosan solution (with the corresponding degree of acetylation) and poloxamer with the reticulating agent and LMWH solution (theoretical load of 25%).

The characteristics of these nanoparticles are represented in Table VI. The size and dispersion of both nanoparticle systems are similar, regardless of the degree of acetylation of the chitosan. However, the zeta potential is less positive for the nanoparticles prepared with the acetylated chitosan due to the fact that part of the amino groups are acetylated. This chemical modification of the chitosan also causes a considerable increase in the production yield and in the percentage of heparin associated to the colloidal system.

Table VI:
Influence of the degree of acetylation of chitosan (LMWCS; <10 kDa) on the formation of nanoparticles with LMWH (theoretical load of 25% with respect to chitosan) and presence of Poloxamer 188 (n≥3; Mean ± SD)

| Degree of acetylation of chitosan (%) | Size (nm) | Polydispersity Index (P.I.) | Zeta Potential (mV) | Association effectiveness (%) | Production yield (%) |
|---|---|---|---|---|---|
| 10-15 | 164 ± 3 | 0.13 | + 26.6 ± 0.8 | 92 | 45.5 ± 3.3 |
| 35-45 | 191 ± 24 | 0.14 | + 12.7 ± 2.0 | 99 | 73.4 ± 5.3 |

**Example 7**

Stability of the nanoparticulate chitosan systems with heparin in simulated gastrointestinal fluids

For the purpose of orally administering the chitosan nanoparticles with LMWH, the stability of the nanoparticulate systems was initially studied from the size and LMWH release point of view. Different nanoparticles were prepared, using acetylated and unacetylated LMWCS for their formulation, and LMWH, with and without Poloxamer 188, added either during nanoparticle preparation or once they are obtained. The proportion of chitosan with respect to the poloxamer added in all the cases is about 10:1. After the preparation of said nanoparticles, the latter were diluted (system:fluid ratio; 1:3) and incubated at 37°C for 15 and 30 minutes. The size of the nanoparticles was measured before and after incubation.

Table VII shows the ratio of Df/Di (diameter after incubation/diameter before incubation) sizes. In said table, it can be seen that the (unacetylated) chitosan nanoparticles with heparin maintain their initial size in gastric fluid and increase their size up to values of 800-1000 nm (Df/Di = 4-5) in intestinal fluid when they incorporate poloxamer (both before and after preparation of the nanoparticles). However, when they do not incorporate poloxamer in the medium these nanoparticles reach values of 3 μm (Df/Di = 14-15) and therefore lose their characteristic nanometric size. This study verifies that the presence of poloxamer in the nanoparticle suspension has a stabilizing effect on the system, preventing its aggregation.

The acetylated chitosan nanoparticles experience a noticeable increase in particle size after their incubation in intestinal medium, exceeding the maximum size (> 3 μm) allowed by the equipment (Zeta Sizer, Nano series, Nano-ZS, Malvern Instruments, UK).

The release of LMWH from the chitosan nanoparticulate systems was measured by means of quantification of the anti-Xa activity in the supernatant (obtained after centrifuging and filtering the samples), after the incubation in simulated gastric and intestinal fluids without enzymes (USP XXVII). Said release was undetectable or less than 1% of the associated amount.

The anti-Xa activity is a measure of the ability of heparin to inhibit or neutralize, through plasma antithrombin III, the activated Factor X (Factor Xa) active coagulating enzyme.

Table VII:
Stability in gastric and intestinal fluids of the LMWCS-LMWH nanoparticles with and without poloxamer 188 in the medium (n≥3; Mean ± SD)

| Formulation | Medium (Simulated) | Time (min at 37°C) | Df / Di |
|---|---|---|---|
| LMWCS-LMWH-Poloxamer | Gastric F. | 15 | 1.00 |
| (after preparation) 10:1 | Intestinal F. | 30 | 4.34 |
| LMWCS-LMWH-Poloxamer | Gastric F. | 15 | 1.00 |
| (added during preparation) 10:1 | Intestinal F. | 30 | 5.15 |
| LMWCS-LMWH | Gastric F. | 15 | 1.03 |
| (Without Poloxamer) | Intestinal F. | 30 | 14.66 |
| LMWCS-Acetylated-LMWH-Poloxamer | Gastric F. | 15 | 1.02 |
| (added in the preparation) 10:1 | Intestinal F. | 30 | > 15 |

Df: Nanoparticle diameter after incubation in the medium; Di: Initial nanoparticle diameter;
LMWCS: Low molecular weight chitosan; LMWH: Low molecular weight heparin

**Example 8**

Chitosan-heparin (both with low molecular weight) nanoparticle lyophilisation study

For the purpose of facilitating preservation of the nanoparticulate systems and preventing possible degradations, a lyophilisation study was conducted on the HMWCS-LMWH system incorporating poloxamer in its preparation, in which different cryoprotectants, such as sucrose, glucose and trehalose, have been tested. The presence of cryoprotectants is indispensable since after lyophilising the formulation (without diluting) without these protectors and reconstituting it with water, polymeric aggregates appear, losing their physicochemical characteristics.

After the preparation of the chitosan nanoparticles with LMWH an amount of the cryoprotectants selected to reach the concentrations of 1 or 5% (w/v) was added. The formulations were metered into vials, frozen (-20°C) and lyophilised (Freeze-Dry System - 12 L, Labconco), in which the first desiccation was carried out at -35°C for 40 hours, and the second desiccation was carried out by raising the temperature (1°C/min) to 0°C (1 hour), then up to 14°C (1 hour) and finally up to 25°C. The lyophilised products were resuspended in water and the size was measured before and after the lyophilisation process. Table VIII shows the ratio of Df/Di (final diameter- lyophilised and resuspended system / initial diameter before lyophilising) sizes, such that values close to 1 indicate that the nanoparticulate system maintains the initial nanometric size after the lyophilisation and resuspension process.

As indicated in the following table, resuspension of the lyophilised system is suitable when adding 5% of the studied cryoprotectants.

Table 8:
LMWCS-LMWH-Poloxamer nanoparticle lyophilisation study (10/1; Polox/CS) with different cryoprotectants (n≥2; Mean ± SD)

| Cryoprotectant | Amount (w/v) | Df/Di |
|---|---|---|
| Sucrose | 1 | 1.16 ± 0.04 |
| | 5 | 1.06 ± 0.07 |
| Trehalose | 1 | 1.26 ± 0.06 |
| | 5 | 1.02 ± 0.03 |

(continued)

LMWCS-LMWH-Poloxamer nanoparticle lyophilisation study (10/1; Polox/CS) with different cryoprotectants (n≥2; Mean ± SD)

| Cryoprotectant | Amount (w/v) | Df/Di |
|---|---|---|
| Glucose | 1 | 1.77 ± 0.23 |
| | 5 | 0.99 ± 0.04 |

Df: Diameter of the nanoparticles after lyophilization; Di: Diameter of the nanoparticles before the lyophilization process.

**Example 9**

Oral administration of low molecular weight heparin in solution and associated to the chitosan nanoparticles (with high molecular weight) in rats. Bioavailability determination.

After the preparation of HMWCS-LMWH nanoparticles according to the process described in Example 1, these nanoparticles were lyophilised in the presence of sucrose (5%) and were reconstituted in a suitable volume of water so as to administer 200 IU/ml/rat. It must be pointed out that heparin is metered in International Units, by reference to a standard, changing the amount of milligrams in relation to the number of Units it has. By definition, an International Unit is equal to one antithrombin unit and one anti-Xa Unit.

The nanoparticles were not isolated for the purpose of eliminating the heparin in solution due to the fact that the amount of heparin associated to the colloidal system exceeded 95%, and for that reason the amount of non-associated heparin was neglected and the total amount of molecule considered upon adjusting the administration volume (according to dose). An LMWH formulation was prepared, which was also lyophilised (at the same concentration and under the same conditions) and resuspended at the same volume as the nanoparticles so that preparations had the same amount of sucrose.

The animals (n=5 for each group) were kept in fasting conditions for 12 hours before the administration and plasma samples were taken at the following times: 0 (pre-administration), 1, 2, 4, 6, 8 and 10 hours.

Anti-Xa activity (IU/ml) was quantified in the plasma samples by means of the use of a colorimetric kit (Stachrom Heparin, Diagnostica Stago, Roche), performing a standard line with the HPBM in solution.

The results obtained *"in vivo"* are represented in Figure 1, where it can be seen that the HMWCS-LMWH nanoparticles give rise to significantly higher plasma levels (specifically 10 times higher) than when heparin is in solution at 6 hours post-administration.

For the purpose of quantifying the relative bioavailability of the LMWH formulations (in solution and associated to the nanoparticles) administered orally with respect to that corresponding to the subcutaneous administration of LMWH dissolved in saline (0.9%), LMWH was administered by both routes using equal doses and the same conditions. The oral bioavailability over the sampling time (0-10 hours) was 5% for the LMWH solution and 8% for the LMWH associated to the HMWCS nanoparticles.

$$\text{Bioavailability (\%)} = \frac{\text{Area under the curve of oral administration plasma levels x I.V dose.}}{\text{Area under the curve of i.v. administration plasma levels x Oral dose}}$$

**Example 10**

Oral administration of low molecular weight heparin in solution and associated to the chitosan nanoparticles (with low molecular weight) in rats. Bioavailability determination

The process of preparing and administering both formulations was exactly the same as that described in the previous example, with the exception that the chitosan used on this occasion is low molecular weight chitosan (Example 5) and in which the plasma sampling range was extended up to 4 hours (every 2 hours).

The anti-Xa activity (IU/ml) plasma levels show that intestinal absorption of the LMWH associated to the LMWCS nanoparticles generates a more prolonged therapeutic response over time, giving significantly higher values between

6 and 12 hours post-administration.

For the purpose of quantifying the bioavailability of both formulations administered orally, the subcutaneous administration of LMWH diluted in saline (0.9%) to a group of rats (equal doses and conditions) was taken into account. Bioavailability over the sampling time (0-12 hours) was 6% for the LMWH solution and 11% for the LMWH associated to the HMWCS nanoparticles.

## Claims

1. A pharmaceutical composition comprising nanoparticles with a size of less than 1 micron for the release of heparin through mucosa, wherein the nanoparticles comprise at least chitosan or a derivative thereof, and at least one heparin or a derivative thereof, and wherein the nanoparticles are crosslinked by means of a crosslinking agent.

2. A composition according to claim 1, wherein the crosslinking agent is a polyphosphate salt, preferably sodium tripolyphosphate.

3. A composition according to claim 1 or 2, wherein the nanoparticles comprise:

    a) between 50% and 90% by weight of chitosan or a derivative thereof, and
    b) between 10% and 50% by weight of heparin or a derivative thereof.

4. A composition according to claim 1, wherein the nanoparticles further comprise a polyoxyethylenated compound.

5. A composition according to claim 4, wherein the polyoxyethylenated compound is a polyoxyethylene or an ethylene oxide-propylene oxide polymer.

6. A composition according to claim 4, wherein the proportion of chitosan or a derivative thereof with respect to the starting amount of the polyoxyethylenated compound is comprised between 50:1 and 1:50 by weight, preferably between 50:1 and 1:20.

7. A composition according to any of the previous claims, wherein the chitosan or a derivative thereof has a molecular weight comprised between 2 and 10000 kDa, preferably between 2 and 500 kDa, more preferably between 5 and 150 kDa.

8. A composition according to any of the previous claims, wherein the chitosan or a derivative thereof has a degree of deacetylation comprised between 30% and 95%, preferably between 55% and 90%.

9. A composition according to any of the previous claims, wherein the crosslinking agent and chitosan ratio is comprised between 0.01:1 and 0.50:1, preferably between 0.05:1 and 0.40:1.

10. A composition according to any of the previous claims, wherein the electric charge (Z potential) of the nanoparticles is comprised between 0 mV and +50 mV, preferably between +1 and +40 mV.

11. A composition according to any of the previous claims for oral administration.

12. A process for preparing a pharmaceutical composition for the administration of heparin by mucosal route according to any of claims 1-11 and comprising:

    a) preparing an aqueous solution comprising chitosan or a derivative thereof;
    b) preparing an aqueous solution comprising heparin or a derivative thereof and the crosslinking agent; and
    c) mixing, with stirring, the solutions of steps a) and b), such that the chitosan-heparin nanoparticles are spontaneously obtained by means of ionic gelling.

13. A process according to claim 12, wherein the aqueous solution of step a) further comprises a polyoxyethylenated compound.

14. A process according to claim 12, wherein the aqueous solution of step a) further comprises inorganic salts, preferably sodium chloride.

**15.** A process according to any of claims 12 to 14, wherein the crosslinking agent is a tripolyphosphate, preferably sodium tripolyphosphate.

FIGURE 1

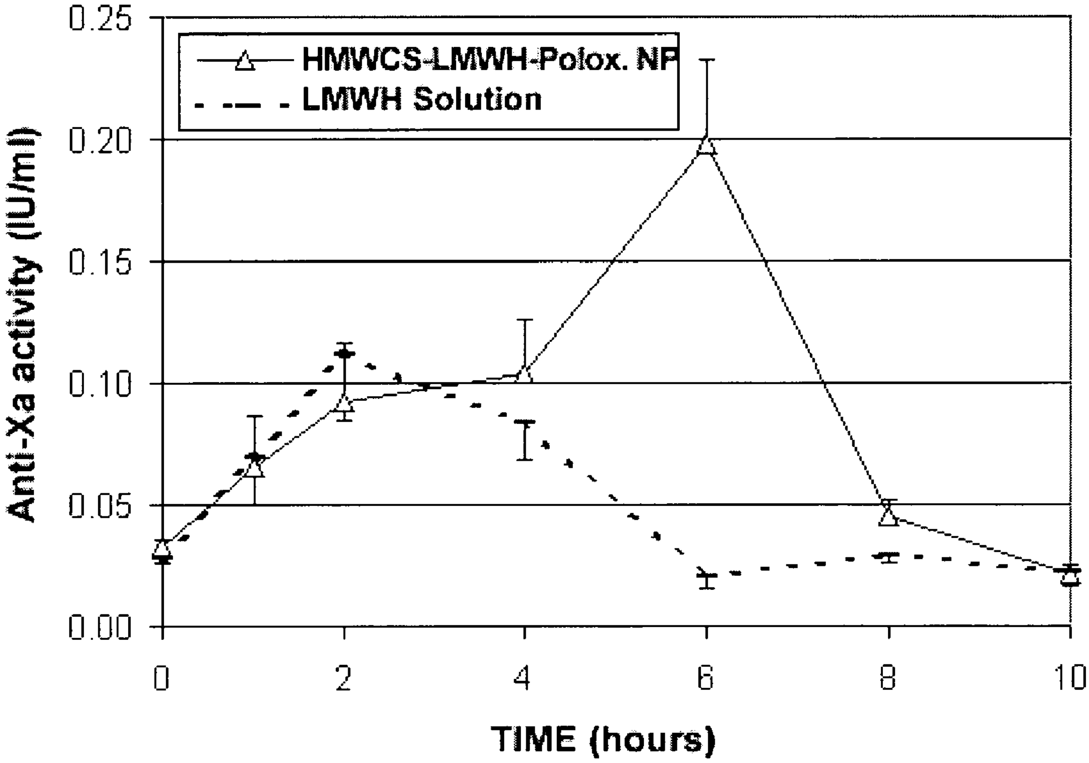
HMWCS-LMWH-Polox. NP
LMWH Solution
Anti-Xa activity (IU/ml)
0.25
0.20
0.15
0.10
0.05
0.00
0
2
4
6
8
10
TIME (hours)

FIGURE 2

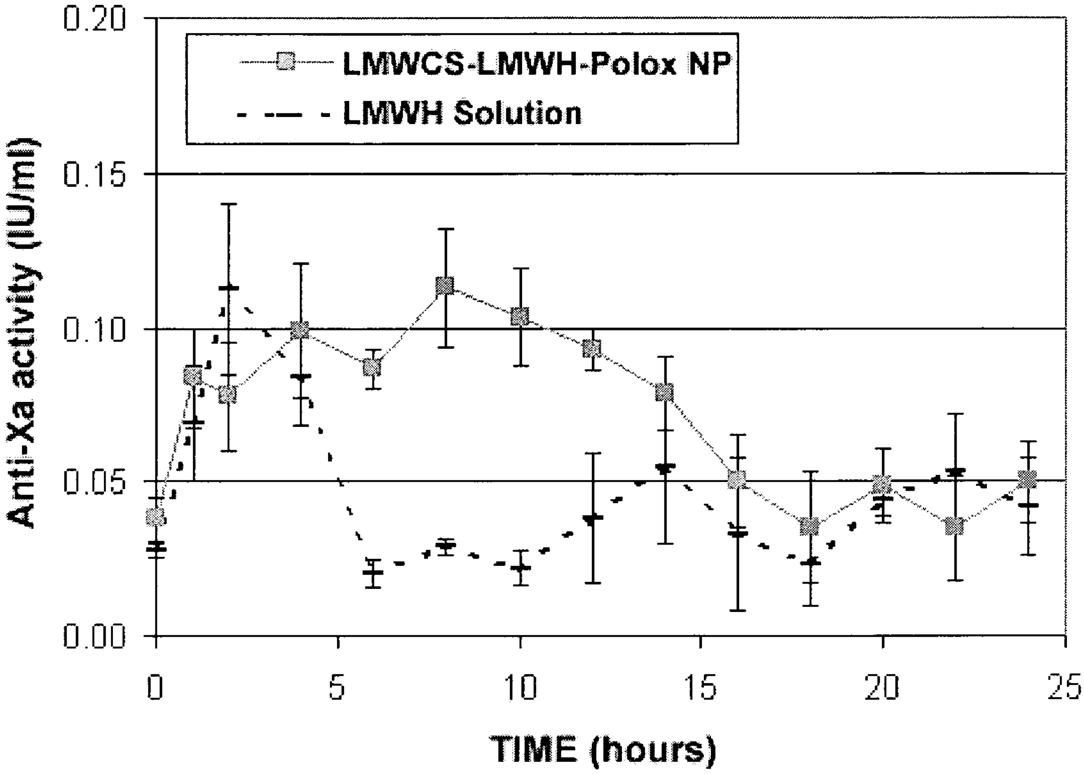

0.20
0.15
0.10
0.05
0.00
Anti-Xa activity (IU/ml)
LMWCS-LMWH-Polox NP
LMWH Solution
0
5
10
15
20
25
TIME (hours)

European Patent Office

# EUROPEAN SEARCH REPORT

Application Number
EP 05 38 0228

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANDERSSON MARTIN ET AL: "Small particles of a heparin/chitosan complex prepared from a pharmaceutically acceptable microemulsion." INTERNATIONAL JOURNAL OF PHARMACEUTICS. 12 MAY 2003, vol. 257, no. 1-2, 12 May 2003 (2003-05-12), pages 305-309, XP002360394 ISSN: 0378-5173 * page 307 - page 308 * ----- | 1-15 | A61K31/722 A61K31/727 A61K33/42 A61K9/51 |
| X | LUBBEN VAN DER I M ET AL: "CHITOSAN AND ITS DERIVATIVES IN MUCOSAL DRUG AND VACCINE DELIVERY" EUROPEAN JOURNAL OF PHARMACEUTICAL SCIENCES, ELSEVIER, AMSTERDAM, NL, vol. 14, no. 3, 2001, pages 201-207, XP001115063 ISSN: 0928-0987 p. 204, item 4 * page 201, right-hand column, last paragraph - page 202, left-hand column, paragraph 1 * ----- | 1-15 | TECHNICAL FIELDS SEARCHED (IPC) A61K |
| X | THANOU M ET AL: "Mono-N-carboxymethyl chitosan (MCC), a polyampholytic chitosan derivative, enhances the intestinal absorption of low molecular weight heparin across intestinal epithelia in vitro and in vivo" JOURNAL OF PHARMACEUTICAL SCIENCES, vol. 90, no. 1, January 2001 (2001-01), pages 38-46, XP002360395 ISSN: 0022-3549 * page 44 - page 45 * ----- -/-- | 1-15 | |

The present search report has been drawn up for all claims

| Place of search | Date of completion of the search | Examiner |
|---|---|---|
| Munich | 2 March 2006 | Zimmer, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

European Patent Office

# EUROPEAN SEARCH REPORT

Application Number
EP 05 38 0228

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 1 561 460 A (ADVANCELL - ADVANCED IN VITRO CELL TECHNOLOGIES) 10 August 2005 (2005-08-10) * claims * ----- | 1-15 | |
| X,D | WO 98/04244 A (UNIVERSIDADE DE SANTIAGO DE COMPOSTELA; ALONSO FERNANDEZ, MARIA JOSE;) 5 February 1998 (1998-02-05) * the whole document * ----- | 1-15 | |
| X | WO 2004/096998 A (VANDERBILT UNIVERSITY) 11 November 2004 (2004-11-11) * claims 1,9,10 * ----- | 1-15 | |
| X | WO 02/41829 A (PR PHARMACEUTICALS, INC; DUNN, JAMES, M) 30 May 2002 (2002-05-30) * page 8, paragraph 1; examples 1-6 * ----- | 1-15 | |
| X | WO 97/04747 A (DUNN, JAMES, M) 13 February 1997 (1997-02-13) * claims 6,16; example 1 * ----- | 1-15 | TECHNICAL FIELDS SEARCHED (IPC) |

The present search report has been drawn up for all claims

| Place of search | Date of completion of the search | Examiner |
|---|---|---|
| Munich | 2 March 2006 | Zimmer, B |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT ON EUROPEAN PATENT APPLICATION NO. EP 05 38 0228

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2006

| Patent document cited in search report | | Publication date | Patent family member(s) | | | Publication date |
|---|---|---|---|---|---|---|
| EP 1561460 | A | 10-08-2005 | CA | 2492995 | A1 | 29-01-2004 |
| | | | WO | 2004009060 | A1 | 29-01-2004 |
| | | | ES | 2221530 | A1 | 16-12-2004 |
| WO 9804244 | A | 05-02-1998 | AT | 285758 | T | 15-01-2005 |
| | | | CA | 2233501 | A1 | 05-02-1998 |
| | | | DE | 69634135 | D1 | 03-02-2005 |
| | | | DE | 69634135 | T2 | 08-12-2005 |
| | | | EP | 0860166 | A1 | 26-08-1998 |
| | | | ES | 2114502 | A1 | 16-05-1998 |
| | | | PT | 860166 | T | 29-04-2005 |
| WO 2004096998 | A | 11-11-2004 | NONE | | | |
| WO 0241829 | A | 30-05-2002 | AU | 3927902 | A | 03-06-2002 |
| | | | CA | 2429254 | A1 | 30-05-2002 |
| WO 9704747 | A | 13-02-1997 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0771206 A
- EP 0930885 A
- EP 0772446 A
- EP 0759760 A
- WO 03090763 A
- WO 9620730 A
- WO 9804244 A
- WO 2004009060 A
- WO 200411275 A
- WO 9605810 A


### Non-patent literature cited in the description


- **ANDERSSON, M. ; LOFROTH, JE.** *Int J Pharm.,* 2003, vol. 257 (1-2), 305-309
- Chitin Chemistry. Macmillan, 1992, 166